Europäisches Patentamt

European Patent Office

Office européen des brevets

(11) Numéro de publication: **0 419 308 A2**

## DEMANDE DE BREVET EUROPEEN

(21) Numéro de dépôt: 90402383.5

(22) Date de dépôt: 29.08.90

(51) Int. Cl.5 **A61K 31/195, A61K 31/725**

Le titre de l'invention a été modifié (Directives relatives à l'examen pratiqué à l'OEB, A-III, 7.3)

(30) Priorité: 30.08.89 FR 8911378

(43) Date de publication de la demande:
27.03.91 Bulletin 91/13

(84) Etats contractants désignés:
GR

(71) Demandeur: **Chevance, Léon Georges**
**1, Avenue Jean Jaurès**
**FR-94340 Joinville Le Pont(FR)**

(72) Inventeur: **Chevance, Léon Georges**
**1, Avenue Jean Jaurès**
**FR-94340 Joinville Le Pont(FR)**

(74) Mandataire: **Clisci, Serge et al**
**S.A. FEDIT-LORIOT & AUTRES 38, avenue Hoche**
**F-75008 Paris(FR)**

(54) Utilisation de l'acide glutamique ou de la gomme arabique comme agent anticomplément.

(57) On utilise comme inhibiteurs du complément dans des médicaments l'acide L-glutamique, le L-glutamate de sodium, la gomme arabique, séparément, en combinaison, ou en association avec d'autres substances thérapeutiques.

EP 0 419 308 A2

# NOUVELLE UTILISATION DE SUBSTANCE COMME ANTICOMPLEMENTS ET MEDICAMENTS RENFERMANT DE TELLES SUBSTANCES

La présente invention concerne l'industrie des médicaments. Elle vise plus particulièrement une nouvelle activité thérapeutique de substances déjà connues en laboratoire ou pour d'autres applications, éventuellement thérapeutiques, différentes.

L'invention vise plus particulièrement une activité jusqu'ici inconnue de l'acide L-glutamique, du L-glutamate de sodium et de la gomme arabique.

L'acide L-glutamique, plus particulièrement sous forme de sel monosodique est connu et largement utilisé comme agent de sapidité dans l'industrie alimentaire. Il est utilisé en thérapeutique pour le traitement du coma hépatique.

La gomme arabique est connue et utilisée comme excipient, épaississeur, stabilisateur colloïdal, fixateur d'arôme dans les industries alimentaire et pharmaceutique, mais on ne lui reconnaissait pas jusqu'ici d'activité thérapeutique particulière.

L'invention a pour objet une nouvelle utilisation de substances du groupe comprenant l'acide L-glutamique, le L-glutamate de sodium, et la gomme arabique, séparément, en combinaison ou en association, dans l'obtention de médicaments destinés à une utilisation en médecine humaine et vétérinaire pour l'inhibition du complément.

L'invention a aussi pour objet les médicaments anti-complément caractérisés en ce qu'ils renferment une proportion active d'au moins une substance du groupe comprenant l'acide L-glutamique, le L-glutamate de sodium et la gomme arabique.

Un médicament suivant l'invention peut renfermer également au moins une autre substance douée d'une activité thérapeutique, et/ou un excipient, et/ou un autre adjuvant.

Un tel médicament peut renfermer avantageusement un mélange synergique de gomme arabique et de L-glutamate monosodique.

L'activité anti-complément des substances visées par l'invention peut être mise en évidence suivant la technique décrite dans l'article de L-G-CHEVANCE et M. ETIEVANT "Etude d'un peptide (sel de magnésium de l' acide N-Acétyl $(\alpha-\beta)$- Aspartyl-glutamique) mise en évidence de la protection contre les destructions cellulaires locales provoquées par l'activation in situ du complément" (J. Pharmacol. Paris 1986 n° 4, 676-685), ainsi que dans la parution de J. RAPP et T. BORSOS "Molecular basis of complement action" Appleton Century Crofts publ. 1970.

Exemples :

Après titrage du complément, on incube à 37°C pendant 45 minutes des prélèvements aliquots de 40 $\mu$l de sérum frais dilué au 1/40e, mélangés chacun respectivement à 40 $\mu$l de solutions aqueuses de concentrations croissantes de chaque substance analysée : acide L-glutamique, glutamate de sodium, gomme arabique et d'un mélange, à concentrations égales de gomme arabique et de glutamate de sodium.

Il n'y a pas d'action hémolytique spontanée, aux concentrations utilisées, sur les érythrocytes sensibilisés.

Les résultats obtenus sont rassemblés dans le tableau suivant :

| Concentration % | % inhibition | | | |
|---|---|---|---|---|
| | Acide glutamique | Glutamate de sodium | Gomme arabique | Mélange gomme arabique/glutamate |
| 2 | 0,661 | - | 0,242 | - |
| 1,5 | 0,369 | - | 0,150 | - |
| 1 | 0,077 | 0,216 | 0,028 | 0,428 |
| 0,5 | 0,092 | 0,175 | 0,031 | 0,368 |
| 0,25 | 0,000 | 0,112 | 0,028 | 0,248 |
| 0,125 | - | 0,116 | - | 0,143 |
| 0,062 | - | 0,101 | - | 0,170 |
| 0,05 | 0,033 | - | 0,000 | - |

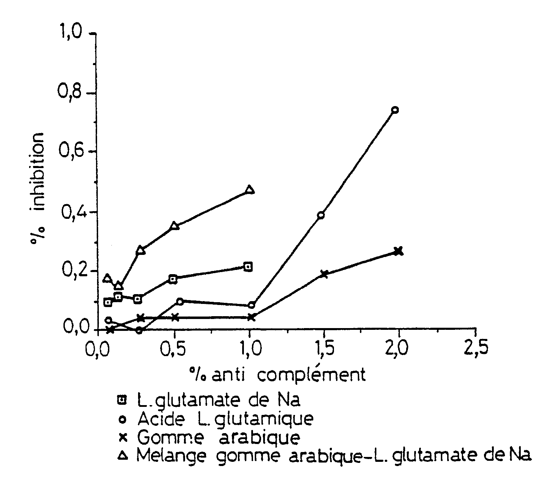

Ce tableau met en évidence une activité synergique du mélange gomme arabique - glutamate de sodium, qui apparaît encore mieux sur la figure unique du dessin annexé qui représente un diagramme schématique rassemblant les données du tableau ci-dessus.

L'invention concerne également un procédé de préparation d'une composition thérapeutique humaine ou vétérinaire pour l'inhibition du complément, ledit procédé étant caractérisé en ce qu'il comprend l'utilisation d'une substance choisie parmi l'ensemble constitué par l'acide L-glutamique, le L-glutamate de sodium, la gomme arabique et leurs mélanges en tant que moyen anticomplément.

## Revendications

1. Utilisation d'une substance choisie parmi l'ensemble constitué par l'acide L-glutamique, le L-glutamate de sodium, la gomme arabique et leurs mélanges pour l'obtention d'un médicament destiné à une utilisation en thérapeutique humaine et vétérinaire pour l'inhibition du complément.

2. Utilisation suivant la revendication 1, caractérisée en ce que le L-glutamate de sodium est le L-glutamate monosodique.

3. Médicament anti-complément, caractérisé en ce qu'il renferme une proportion active d'au moins une substance choisie parmi l'ensemble constitué par l'acide L-glutamique, le L-glutamate de sodium, la gomme arabique et leurs mélanges.

4. Médicament anti-complément suivant la revendication 3, caractérisé en ce que le L-glutamate de sodium est le L-glutamate monosodique.

5. Médicament anti-complément suivant la revendication 3, caractérisé en ce qu'il renferme un mélange synergique de gomme arabique et de L-glutamate monosodique.

6. Procédé de préparation d'une composition thérapeutique humaine ou vétérinaire pour l'inhibition du complément, ledit procédé étant caractérisé en ce qu'il comprend l'utilisation d'une substance choisie parmi l'ensemble constitué par l'acide L-glutamique, le L-glutamate de sodium, la gomme arabique et leurs mélanges en tant que moyen anticomplément.

3

% inhibition

% anti complément

□ L.glutamate de Na
o Acide L.glutamique
× Gomme arabique
△ Melange gomme arabique-L. glutamate de Na